(19) Europäisches Patentamt
European Patent Office
Office européen des brevets

(11) Veröffentlichungsnummer: **0 121 944**
**B1**

(12) # EUROPÄISCHE PATENTSCHRIFT

(45) Veröffentlichungstag der Patentschrift: **07.10.87**

(51) Int. Cl.⁴: **C 12 Q 1/26**, G 01 N 33/02

(21) Anmeldenummer: **84104074.4**

(22) Anmeldetag: **11.04.84**

(54) **Verfahren zur enzymatischen Bestimmung von Sulfit.**

(30) Priorität: **12.04.83 DE 3313178**

(43) Veröffentlichungstag der Anmeldung: **17.10.84 Patentblatt 84/42**

(45) Bekanntmachung des Hinweises auf die Patenterteilung: **07.10.87 Patentblatt 87/41**

(84) Benannte Vertragsstaaten: **AT BE CH DE FR GB IT LI LU NL SE**

(56) Entgegenhaltungen:
**DE - A - 2 633 728**

**TRENDS IN ANALYTICAL CHEMISTRY, Band 2, Nr. 1, Januar 1983, Cambridge, GB; N.T. CROSBY "Enzymes in food analysis", Seiten VII,VIII**
**M. DIXON, E.C. WEBB "Enzymes", 3. Auflage 3. Auflage 1979, LONGMAN GROUP LTD., London, GB Seiten 736-737**

**Die Akte enthält technische Angaben, die nach dem Eingang der Anmeldung eingereicht wurden und die nicht in dieser Patentschrift enthalten sind.**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Beutler, Hans-Otto, Dr.rer.nat., Zugspitzstrasse 28, D-8132 Tutzing (DE)**
Erfinder: **Schütte, Ingrid, Kirchstrasse 14, D-8121 Eberfing (DE)**
Erfinder: **Schneider, Walter, Geistbühelstrasse 27, D-8120 Weilheim (DE)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft ein Verfahren und ein Reagenz zur enzymatischen Bestimmung von Sulfit.

In der Lebensmitteltechnologie wird Sulfit wegen seiner bakteriziden und fungiziden und wegen seiner enzyminhibierenden Eigenschaften sehr häufig eingesetzt. Bei Obst- und Gemüseerzeugnissen und bei Obstsäften wird es als Konservierungsmittel verwendet. Darüberhinaus hemmt Sulfit die Maillard-Reaktion und wird deshalb bei der Herstellung von Kartoffelprodukten zur Verhinderung der Braunfärbung eingesetzt. Bei der Weinherstellung ist das «Schwefeln» schon seit Alters her eine kellereiwirtschaftliche Massnahme, die dazu dient, den Wein vor dem Braunwerden und der Entwicklung pathogener Mikroorganismen zu schützen und entstehenden Acetaldehyd abzufangen, um die Essigsäurebildung zu vermeiden. Weiterhin verhindert Sulfit als Inhibitor für viele Enzyme unerwünschte enzymatische Reaktionen.

Obwohl daher die technologische Bedeutung von Sulfit insbesondere auf dem Lebensmittelgebiet sehr gross ist, wird die allgemeine Anwendung dieses Stoffes jedoch begrenzt durch seine toxischen Eigenschaften. Die gesundheitsschädigenden Eigenschaften von Sulfit erfordern eine genaue Kontrolle der Sulfitmenge in dem jeweiligen Lebensmittel. Wegen der gesundheitsschädigenden Eigenschaften sind bestimmte Höchstmengen für Sulfit in Lebensmitteln gesetzlich festgelegt. Daraus ergibt sich die Notwendigkeit, in vielen Lebensmitteln die Sulfitkonzentration zu bestimmen.

Die bisher bekannten Methoden zur Bestimmung von Sulfit, insbesondere in Lebensmitteln sind sehr unbefriedigend. Als Schnelltest wird in lebensmittelchemischen Labors eine jodometrische Bestimmung durchgeführt, bei der jedoch nicht nur Sulfit sondern alle reduzierenden Substanzen miterfasst werden. Diese Methode ist sehr unspezifisch, erfordert jedoch einen relativ geringen Zeitaufwand und wird deswegen bei Routineuntersuchungen bevorzugt. Spezifischer ist eine in der BRD als amtliche Methode für die Untersuchung von Wein vorgeschriebene Sulfitbestimmung, bei der durch Erhitzen mit Phosphorsäure die schwefelige Säure freigesetzt und in eine mit Wasserstoffperoxidlösung beschickte Vorlage überdestilliert wird, wobei die schwefelige Säure zu Sulfat oxidiert und als Sulfat bestimmt wird. Diese Methode erfordert jedoch einen sehr grossen Zeitaufwand.

Aufgabe der Erfindung war es daher, ein Verfahren zur Bestimmung des Sulfits zu schaffen, das sowohl spezifisch für Sulfit als auch mit geringem Zeitaufwand durchzuführen ist.

Gelöst wird diese Aufgabe durch ein Verfahren zur Bestimmung von Sulfit, das dadurch gekennzeichnet ist, dass man das Sulfit in einer gepufferten Lösung mit Sulfitoxidase zu Sulfat oxidiert, das dabei entstehende $H_2O_2$ in Gegenwart von NAD und NADH mit NADH-Peroxidase reduziert und den Verbrauch an NADH optisch bestimmt.

Es war überraschend, dass eine enzymatische Methode zur Bestimmung des Sulfits gefunden werden konnte, da einerseits zu befürchten war, dass das bei der Redox-Reaktion gebildete $H_2O_2$ sofort mit dem noch in der Lösung vorhandenen reduzierend wirkenden Sulfit reagieren würde, was das Ergebnis verfälschen würde und andererseits eine bekannte Eigenschaft des Sulfits die Inhibierung von Enzymen ist, so dass eine enzymatische Bestimmung des entstandenen $H_2O_2$ undurchführbar erscheinen musste. Auch ein Nachweis des entstandenen $H_2O_2$ in üblicher Weise über eine Farbstoffbildung in Gegenwart von Peroxidase erschien aussichtslos, da in der oxidierten Form vorliegender Farbstoff mit noch vorhandenem Sulfit zu in reduzierter Form vorliegendem Leukofarbstoff reagiert und Peroxidase von Sulfit gehemmt wird. Die DE-A-2 633 728 beschreibt Verfahren zum Nachweis von $H_2O_2$ durch Verwendung einer NADH-Peroxidase. Die Verwendung des speziellen Enzyms NADH-Peroxidase E.C. 1.11.1.1. wird dort jedoch in gekoppelten Reaktionen mit einer spezifischen Oxidase als nicht geeignet bezeichnet. Mit Hilfe des erfindungsgemässen Verfahrens ist es nun überraschenderweise möglich, eine enzymatische Methode zur Bestimmung des Sulfits zu schaffen, mit der Sulfit spezifisch und schnell bestimmt werden kann.

Das erfindungsgemässe Verfahren läuft in zwei Stufen ab. Zuerst wird das in einer gepufferten Lösung vorliegende Sulfit mit Hilfe des Enzyms Sulfitoxidase ($SO_2$-OD) EC 1.8.3.1. in Gegenwart von Sauerstoff zu Sulfat oxidiert nach der Gleichung 1):

1)

$$SO_3^{--} + O_2 + H_2O \xrightarrow{SO_2\text{-OD}} SO_4^{--} + H_2O_2$$

Das gebildete Wasserstoffperoxid reagiert in Gegenwart von NAD sofort mit NADH und NADH-Peroxidase EC 1.11.1.1 unter Bildung von Wasser nach der Gleichung 2):

2)

$$H_2O_2 + NADH + H^+ \xrightarrow{NADH\text{-}POD} 2\,H_2O + NAD^+.$$

NADH wirkt dabei als Reduktionsmittel und wird zu $NAD^+$ oxidiert. Da NADH und $NAD^+$ bei verschiedenen Wellenlängen absorbieren, kann die Abnahme von NADH optisch ausgewertet werden. Die bei der Reaktion verbrauchte NADH-Menge ist der Sulfitmenge in der Probelösung äquivalent. NADH kann leicht quantitativ bestimmt werden aufgrund seiner Absorption z.B. bei 365, 340 oder 334 nm.

Die Reaktion wird in einer gepufferten Lösung durchgeführt, deren pH im Bereich von 7,5 bis 8,5 liegt. Bevorzugt ist der pH-Bereich von 7,8 bis 8.2.

Für die Sulfitbestimmung sind diverse Puffersubstanzen geeignet. Bevorzugt werden Glycylglycin-, Imidazol-, Tris- und Triäthanolamin-Puffer. Besonders bevorzugt wird Triäthanolamin-Puffer. Die Konzentration des Puffers hat auf den Testablauf keinen grossen Einfluss. Als besonders geeignet erwies sich eine Konzentration des Puffers im Bereich von 0,05 bis 0,4 Mol/l. Dabei wird der Bereich von 0,1 bis 0,3 Mol/l besonders bevorzugt.

Die für die Bestimmung erforderliche Menge an NADH sollte nicht zu hoch sein, damit die Extinktion für den Anfangswert im gut messbaren Bereich bleibt, andererseits sollte sie auch nicht zu niedrig sein, damit die Reaktion noch quantitativ ablaufen kann. Als zweckmässig erwies sich eine Konzentration von NADH im Bereich von 100 bis 500 µmol/l. Besonders geeignet ist eine Menge von 125 bis 225 µmol/l NADH.

Ferner wird der Probelösung NAD zugesetzt. Eine Menge von 0,5 bis 2,0 mMol/l NAD erwies sich als ausreichend, obwohl grössere Mengen verwendbar sind. Besonders geeignet ist der Zusatz von 0,75 bis 1,00 mMol/l NAD.

Die Menge an NADH-Peroxidase ist unkritisch. Setzt man jedoch zu wenig Aktivität ein, so wird die Reaktionszeit lang und die Sulfitwiederfindung gelegentlich zu gering. Es sollten deshalb zweckmässigerweise mindestens 10 U/l NADH-Peroxidase in der Probelösung eingesetzt werden. Mengen über 100 U/l NADH-Peroxidase führen zu keiner weiteren Verminderung der Reaktionszeit und sind daher unwirtschaftlich. Als zweckmässig hat sich deshalb eine Konzentration der NAD-Peroxidase von 10 bis 100 U/l erwiesen. Besonders bevorzugt wird die NADH-Peroxidase in einer Menge von 40 bis 80 U/l eingesetzt.

Auch die Menge des für die Oxidation des Sulfits zum Sulfat notwendigen Enzyms Sulfitoxidase ist nicht kritisch. Jedoch erwies sich eine Aktivität von weniger als 25 U/l Sulfitoxidase als weniger günstig, da dann die Reaktionszeit lang wird und die Wiederfindung abfällt. Dabei wird zweckmässig die Sulfitoxidase in einer Menge von 25 bis 75 U/l eingesetzt. Eine höhere Aktivität an Sulfitoxidase ist möglich aber unwirtschaftlich, da sie keine weiteren Verbesserungen bringt. Besonders bevorzugt wird die Sulfitoxidase in einem Bereich von 35 bis 60 U/l eingesetzt.

Das erfindungsgemässe Verfahren ist spezifisch für Sulfit. Grössere Mengen an Ascorbinsäure im Probematerial können jedoch den Test stören. Da Sulfit insbesondere in Obst- und Gemüseerzeugnissen und in Fruchtsäften nachgewiesen werden muss, die Ascorbinsäure in mehr oder minder grossen Mengen enthalten, erwies es sich als zweckmässig, die Ascorbinsäure vor der Analyse zu entfernen. Ein geeignetes Verfahren zur Entfernung der Ascorbinsäure ist ein Zusatz von Ascorbatoxidase zu der Probelösung gemäss DE-OS 26 25 834.4.

Ein weiterer Gegenstand der Erfindung ist ein Reagenz zur Bestimmung von Sulfit, das dadurch gekennzeichnet ist, dass es Sulfitoxidase, NADH-Peroxidase, NAD, NADH sowie Puffer enthält.

Dieses Reagenz ermöglicht einen einfachen, spezifischen und schnellen Nachweis von Sulfit.

Das Reagenz enthält im gebrauchsfertigen Zustand vorzugsweise 0,05 bis 0,4 Mol/l Puffer mit einem pH-Wert-Bereich von 7,5 bis 8,5, 100 bis 500 µmol/l NADH, 0,5 bis 2,0 mMol/l NAD, 10 bis 100 U/l NADH-Peroxidase und 25 bis 75 U/l Sulfitoxidase bzw. die entsprechenden Mengen im Trockenreagenz. Für Ascorbinsäure-haltige Probelösungen ist ausserdem noch ein Gehalt von 10 bis 50 U/l Ascorbatoxidase zweckmässig.

Besonders bevorzugt enthält das Reagenz 0,1 bis 0,3 Mol Puffer, 125 bis 225 µMol NADH, 0,75 bis 1,0 mMol NAD, 40 bis 80 U NADH-Peroxidase und 35 bis 60 U Sulfitoxidase, bezogen auf 1 Liter gebrauchsfertige Lösung.

Das erfindungsgemässe Verfahren und Reagenz zur Bestimmung von Sulfit zeichnet sich aus durch seine Spezifität gegenüber Sulfit, durch eine einfache Versuchsdurchführung und geringen Zeitaufwand. Somit wird es möglich, den Sulfitgehalt schnell und quantitativ zu bestimmen, ohne dass andere reduzierende Substanzen die Ergebnisse verfälschen können. Das erfindungsgemässe Verfahren und Reagenz ist für Routineanalysen besonders geeignet. Es eignet sich sowohl für die Bestimmung des Sulfits in Lebensmitteln als auch im Abwasser oder in der Luft. Es kann auch an Trägersubstanzen gebunden, z.B. in Form von Teststreifen angewendet werden.

Die folgenden Beispiele erläutern die Erfindung.

Beispiel 1

Es wurde Sulfit in verschiedenen Lebensmitteln bestimmt. Dazu werden nacheinander in eine Glasküvette mit 1 cm Schichtdicke folgende Komponenten pipettiert:

Komponente 1: 2 mg NAD, 0,4 mg NADH, gelöst in 1 ml Triäthanolamin-Puffer mit pH 8,0;

Komponente 2: 0,01 ml Enzymsuspension, enthaltend 0,1 U NADH-Peroxidase,

1,9 ml bidestilliertes Wasser und 0,1 ml Probelösung.

In eine zweite Küvette werden die Komponenten 1 und 2 und das bidestillierte Wasser in denselben Mengen wie bei der ersten Küvette pipettiert und zusätzlich 0,1 ml bidestilliertes Wasser anstelle der Probelösung zugegeben. In beiden Küvetten wird die Reaktionslösung gemischt und nach ca. 5 Minuten werden die Extinktionen der Lösungen gemessen. Dies ergibt den Extinktionswert $E_1$. Nach der Messung wird die Reaktion gestartet durch Zugabe von

Komponente 3: 0,05 ml Enzymsuspension, enthaltend 0,13 U Sulfitoxidase,

zu jeder der beiden Küvetten. Es wird wieder gemischt und nach Stillstand der Reaktion, der nach etwa 15 bis 20 Minuten eintritt, werden die Extinktionen der beiden Lösungen gemessen. Es ergibt sich der Extinktionswert $E_2$. Die Konzentration des Sulfits in der Probe lässt sich dann nach der folgenden allgemeinen Berechnungsformel berechnen:

$$c = \frac{V \times MG}{\varepsilon \times d \times v \times 1000} \times \Delta E \text{ [g/1,]},$$

wobei

$V$ = Testvolumen (ml),
$v$ = Probevolumen (ml),
MG = Molekulargewicht der zu bestimmenden Substanz,
$d$ = Schichtdicke (cm),
$\varepsilon$ = Extinktionskoeffizient von NADH und
$\Delta\varepsilon = (E_1-E_2)_{Probe} - (E_1-E_2)_{Leerwert}$

Die Messung kann durchgeführt werden bei verschiedenen Wellenlängen. Für die Messung bei 340 nm ergibt sich für Sulfit folgende Formel:

$$c = 1{,}960 \times \frac{\Delta E}{6{,}3} \text{ [g } SO_2\text{/1 Probelösung]}.$$

Ist bei der Probenvorbereitung eine Verdünnung vorgenommen worden, so muss diese noch bei dem Ergebnis berücksichtigt werden.

Beispiel 2

Es wurde der Gesamt-$SO_2$-Gehalt in Weisswein bestimmt. Dazu wurde 0,1 ml des zu untersuchenden Weissweins als Probevolumen direkt zum Test eingesetzt. Die Untersuchung wurde durchgeführt, wie im Beispiel 1 beschrieben.

Beispiel 3

Es wurde eine Sulfitbestimmung in Rotwein durchgeführt. Dazu wurden 25 ml Rotwein mit Natronlauge (2 Mol/l) auf pH 7,5 bis 8,0 eingestellt. Die Lösung wurde mit bidestilliertem Wasser auf 50 ml verdünnt und 10 Minuten bei Raumtemperatur stehen gelassen. Von dieser Lösung wurden 0,1 ml als Probevolumen zum Test eingesetzt. Die Bestimmung wurde durchgeführt, wie im Beispiel 1 beschrieben.

Beispiel 4

Es wurde eine Sulfitbestimmung in Fruchtsaft durchgeführt. Um die Ascorbinsäure aus dem Saft zu entfernen, wurden 2,0 ml des Fruchtsaftes mit Natronlauge (2 Mol/l) auf pH 5 bis 6 eingestellt und mit ca. 20 U Ascorbatoxidase-Lösung versetzt, gemischt und 10 Minuten stehen gelassen. Danach wurde die Probe mit Natronlauge auf pH 7,5 bis 8,0 eingestellt. Die Lösung wurde auf 4,0 ml mit bidestilliertem Wasser verdünnt. Von dieser verdünnten Lösung wurden 0,2 ml als Probelösung zum Test eingesetzt. Die Durchführung des Tests erfolgte, wie im Beispiel 1 beschrieben.

Beispiel 5

Es wurde Sulfit in einer Konfitüre bestimmt. Dazu wurden etwa 100 g Konfitüre in einem Homogenisator 30 Sekunden lang homogenisiert. 5 g dieser homogenisierten Probe wurden in einen 50 ml Messkolben eingewogen und mit 40 ml Wasser aufgefüllt. Der Messkolben wurde verschlossen und 5 Minuten auf 60°C erhitzt. Nach dem Abkühlen auf Raumtemperatur wurde mit bidestilliertem Wasser bis zur Marke aufgefüllt, gemischt und filtriert. Von der klaren Lösung wurden 0,2 ml als Probevolumen zum Test eingesetzt. Die Bestimmung erfolgte, wie im Beispiel 1 beschrieben.

Beispiel 6

Es wurde Sulfit in einem Kartoffelerzeugnis bestimmt.

Dazu wurden etwa 5 g zerkleinerte und gemahlene Kartoffelchips in einem 50 ml Messkolben eingewogen. Es wurde mit 40 ml bidestilliertem Wasser aufgefüllt. Der Messkolben wurde verschlossen und 5 Minuten auf ca. 60°C erhitzt. Nach dem Abkühlen auf Raumtemperatur wurde mit bidestilliertem Wasser bis zur Marke aufgefüllt, gemischt und zentrifugiert. 0,5 ml der klaren Lösung wurden als Probevolumen zum Test eingesetzt. Die Bestimmung erfolgte, wie im Beispiel 1 beschrieben.

Beispiel 7

Es wurde Sulfit in einer Gewürzprobe bestimmt.

Dazu wurde das Gewürz zerkleinert und gemischt. Von der zerkleinerten und gemahlenen Probe wurden ca. 100 mg in einem 50 ml Messkolben eingewogen und 20 ml bidestilliertes Wasser hinzugefügt. Der Messkolben wurde verschlossen und 5 Minuten bei ca. 60°C gehalten. Nach dem Abkühlen auf Raumtemperatur wurde mit bidestilliertem Wasser bis zur Marke aufgefüllt, gemischt und filtriert. 0,1 ml der klaren Lösung wurden als Probevolumen zum Test eingesetzt. Die Durchführung des Tests erfolgte, wie im Beispiel 1 beschrieben.

**Patentansprüche**

1. Verfahren zur enzymatischen Bestimmung von Sulfit, dadurch gekennzeichnet, dass man das Sulfit in gepufferter Lösung mit Sulfitoxidase zu Sulfat oxidiert, das dabei entstehende $H_2O_2$ in Gegenwart von NAD und NADH mit dem Enzym NADH-Peroxidase E.C. 1.11.1.1. reduziert und den Verbrauch an NADH optisch bestimmt.

2. Verfahren nach Anspruch 1, dadurch gekennzeichnet, dass man eine Pufferlösung mit einem pH-Wert von 7,5 bis 8,5 verwendet.

3. Verfahren nach Anspruch 2, dadurch gekennzeichnet, dass man eine Pufferlösung mit einem pH-Wert von 7,8 bis 8,2 verwendet.

4. Verfahren nach einem der vorhergehenden Ansprüche, dadurch gekennzeichnet, dass man Triäthanolaminpuffer verwendet.

5. Verfahren nach einem der vorhergehenden Ansprüche, dadurch gekennzeichnet, dass man Puffer in einer Konzentration von 0,05 bis 0,4 Mol/l verwendet.

6. Verfahren nach einem der vorhergehenden Ansprüche, dadurch gekennzeichnet, dass man NADH in einer Konzentration von 100 bis 500 µmol/l verwendet.

7. Verfahren nach einem der vorhergehenden

Ansprüche, dadurch gekennzeichnet, dass man 0,5 bis 2,0 mMol/l NAD einsetzt.

8. Verfahren nach einem der vorhergehenden Ansprüche, dadurch gekennzeichnet, dass man 10 bis 100 U/l NADH-Peroxidase einsetzt.

9. Verfahren nach einem der vorhergehenden Ansprüche, dadurch gekennzeichnet, dass man 25 bis 75 U/l Sulfitoxidase einsetzt.

10. Verfahren nach einem der vorhergehenden Ansprüche, dadurch gekennzeichnet, dass man vor Durchführung der Sulfitbestimmung Ascorbinsäure aus der Probelösung entfernt.

11. Verfahren nach Anspruch 10, dadurch gekennzeichnet, dass man zur Entfernung der Ascorbinsäure Ascorbatoxidase verwendet.

12. Reagenz zur enzymatischen Sulfitbestimmung, dadurch gekennzeichnet, dass es Sulfitoxidase, NADH, NAD, NADH-Peroxidase E.C. 1.11.1.1. und Puffer enthält.

13. Reagenz nach Anspruch 12, dadurch gekennzeichnet, dass es bezogen auf 1 Liter gebrauchsfertige Reagenzlösung 0,05 bis 0,4 Mol Puffer, 100 bis 500 µmol NADH, 0,5 bis 2,0 mMol NAD, 10 bis 100 U NADH-Peroxidase und 25 bis 75 U Sulfitoxidase enthält.

14. Reagenz nach Anspruch 13, dadurch gekennzeichnet, dass es Triäthanolamin-Puffer enthält.

15. Reagenz nach einem der Ansprüche 13 bis 14, dadurch gekennzeichnet, dass es bezogen auf 1 Liter gebrauchsfertige Reagenzlösung 0,1 bis 0,3 Mol Puffer, 125 bis 225 µMol/l NADH, 0,75 bis 1,0 mMol NAD, 40 bis 80 U NADH-Peroxidase und 35 bis 60 U Sulfitoxidase enthält.

16. Reagenz nach einem der Ansprüche 13 bis 15, dadurch gekennzeichnet, dass es zusätzlich Ascorbatoxidase enthält.

**Claims**

1. Process for the enzymatic determination of sulphite, characterised in that one oxidises the sulphite in buffered solution with sulphite oxidase to sulphate, reduces the $H_2O_2$ thereby formed in the presence of NAD and NADH with the enzyme NADH peroxidase E.C. 1.11.1.1 and optically determines the consumption of NADH.

2. Process according to claim 1, characterised in that one uses a buffer solution with a pH value of 7.5 to 8.5.

3. Process according to claim 2, characterised in that one uses a buffer solution with a pH value of 7.8 to 8.2.

4. Process according to one of the preceding claims, characterised in that one uses triethanolamine buffer.

5. Process according to one of the preceding claims, characterised in that one uses buffer in a concentration of 0.05 to 0.4 mol/l.

6. Process according to one of the preceding claims, characterised in that one uses NADH in a concentration of 100 to 500 µmol/l.

7. Process according to one of the preceding claims, characterised in that one uses 0.5 to 2.0 mMol/l. NAD.

8. Process according to one of the preceding claims, characterised in that one uses 10 to 100 U/l. NADH peroxidase.

9. Process according to one of the preceding claims, characterised in that one uses 25 to 75 U/l. sulphite oxidase.

10. Process according to one of the preceding claims, characterised in that one removes ascorbic acid from the sample solution before carrying out the sulphite determination.

11. Process according to claim 10, characterised in that one uses ascorbate oxidase for the removal of the ascorbic acid.

12. Reagent for the enzymatic determination of sulphite, characterised in that it contains sulphite oxidase, NADH, NAD, NADH peroxidase E.C. 1.11.1.1 and buffer.

13. Reagent according to claim 12, characterised in that, referred to 1 litre on reagent solution ready for use, it contains 0.05 to 0.4 mole of buffer, 100 to 500 µmole NADH, 0.5 to 2.0 mMole NAD, 10 to 100 U NADH peroxidase and 25 to 75 U sulphite oxidase.

14. Reagent according to claim 13, characterised in that it contains triethanolamine buffer.

15. Reagent according to one of claims 13 to 14, characterised in that, referred to 1 litre of reagent solution ready for use, it contains 0.1 to 0.3 mole of buffer, 125 to 225 µmole/l. NADH, 0.75 to 1.0 mMole NAD, 40 to 80 U NADH peroxidase and 35 to 60 U sulphite oxidase.

16. Reagent according to one of claims 13 to 15, characterised in that it additionally contains ascorbate oxidase.

**Revendications**

1. Procédé de détermination enzymatique du sulfite, caractérisé en ce qu'on oxyde le sulfite en sulfate en solution tamponnée, avec de la sulfite-oxydase, on réduit l'$H_2O_2$ alors formé en présence de NAD et de NADH avec l'enzyme NADH-peroxydase E.C. 1.11.1.1. et on détermine optiquement la consommation de NADH.

2. Procédé suivant la revendication 1, caractérisé en ce qu'on utilise une solution tampon ayant un pH de 7,5 à 8,5.

3. Procédé suivant la revendication 2, caractérisé en ce qu'on utilise une solution tampon ayant un pH de 7,8 à 8,2.

4. Procédé suivant l'une des revendications précédentes, caractérisé en ce qu'on utilise du tampon triéthanolamine.

5. Procédé suivant l'une des revendications précédentes, caractérisé en ce qu'on utilise le tampon à une concentration de 0,05 à 0,4 mole/l.

6. Procédé suivant l'une des revendications précédentes, caractérisé en ce qu'on utilise le NADH à une concentration de 100 à 500 µmoles/l.

7. Procédé suivant l'une des revendications précédentes, caractérisé en ce qu'on utilise 0,5 à 2,0 mmoles/l de NAD.

8. Procédé suivant l'une des revendications précédentes, caractérisé en ce qu'on utilise 10 à 100 U/l de NADH-peroxydase.

9. Procédé suivant l'une des revendications précédentes, caractérisé en ce qu'on utilise 25 à 75 U/l de sulfite-oxydase.

10. Procédé suivant l'une des revendications précédentes, caractérisé en ce qu'on élimine l'acide ascorbique de la solution d'échantillon avant l'exécution de la détermination du sulfite.

11. Procédé suivant la revendication 10, caractérisé en ce qu'on utilise pour l'élimination de l'acide ascorbique, de l'ascorbate-oxydase.

12. Réactif pour la détermination enzymatique du sulfite, caractérisé en ce qu'il contient de la sulfite-oxydase, du NADH, du NAD, de la NADH-peroxydase E.C. 1.11.1.1 et un tampon.

13. Réactif suivant la revendication 12, caractérisé en ce qu'il contient, pour un litre de solution de réactif prête à l'emploi, 0,05 à 0,4 mole de tampon, 100 à 500 µmoles de NADH, 0,5 à 2,0 mmoles de NAD, 10 à 100 U de NADH-peroxydase et 25 à 75 U de sulfite-oxydase.

14. Réactif suivant la revendication 13, caractérisé en ce qu'il contient du tampon triéthanolamine.

15. Réactif suivant l'une des revendications 13 à 14, caractérisé en ce qu'il contient pour un litre de solution de réactif prête à l'emploi, 0,1 à 0,3 mole de tampon, 125 à 225 µmoles/l de NADH, 0,75 à 1,0 mmole de NAD, 40 à 80 U de NADH-peroxydase et 35 à 60 U de sulfite-oxydase.

16. Réactif suivant l'une des revendications 13 à 15, caractérisé en ce qu'il contient en outre de l'ascorbate-oxydase.